# EUROPÄISCHE PATENTSCHRIFT

(11) **EP 3 412 276 B1**
(45) Veröffentlichungstag und Bekanntmachung des Hinweises auf die Patenterteilung: **19.11.2025**
(21) Anmeldenummer: 18176815.1
(22) Anmeldetag: 08.06.2018
(51) Int. Cl.: A61K 9/00, A61K 47/02, A61K 47/06, A61K 47/10, A61K 47/18, A61K 47/20, A61K 47/22, A61K 47/26, A61K 47/36, A61K 31/00

(54) **ZUSAMMENSETZUNG ZUR BEHANDLUNG DES TROCKENEN AUGES**
COMPOSITION FOR THE TREATMENT OF DRY EYE
COMPOSITION DE TRAITEMENT DE LA SÉCHERESSE OCULAIRE

(30) Priorität: 09.06.2017 DE 202017103487 U; 23.06.2017 DE 202017103767 U
(43) Veröffentlichungstag der Anmeldung: 12.12.2018
(73) Patentinhaber: Omnivision GmbH, 82178 Puchheim (DE)
(72) Erfinder: KOHL, Tobias, 82178 Puchheim (DE); HOFFMANN, Burkhardt, 82178 Puchheim (DE); HOFFMANN, Patrick, 82178 Puchheim (DE)
(74) Vertreter: Wächtershäuser & Hartz Patentanwaltspartnerschaft mbB

(56) Entgegenhaltungen:
- EP-A1- 2 664 330
- EP-A1- 2 783 695
- WO-A1-2008/077712
- AU-A1- 2015 331 089
- DE-A1- 19 511 322
- US-A1- 2014 221 309
- US-A1- 2016 199 428
- WEI CHEN ET AL: "Efficacy of Osmoprotectants on Prevention and Treatment of Murine Dry Eye", INVESTIGATIVE OPTHALMOLOGY & VISUAL SCIENCE, vol. 54, no. 9, 19 September 2013 (2013-09-19), US, pages 6287, XP055511297, ISSN: 1552-5783, DOI: 10.1167/iovs.13-12081
- LIDICH NINA ET AL: "Structural characteristics of oil-poor dilutable fish oil omega-3 microemulsions for ophthalmic applications", JOURNAL OF COLLOID AND INTERFACE SCIENCE, ACADEMIC PRESS,INC, US, vol. 463, 5 November 2015 (2015-11-05), pages 83 - 92, XP029313623, ISSN: 0021-9797, DOI: 10.1016/J.JCIS.2015.10.024
- H SELEK ET AL: "Evaluation of retinoic acid ophthalmic emulsion in dry eye", EUROPEAN JOURNAL OF OPHTHALMOLOGY, 1 April 2000 (2000-04-01), United States, pages 121 - 127, XP055511711, Retrieved from the Internet <URL:https://pdfs.semanticscholar.org/6a49/c9e6b77268eabee97ddcc273299e3ed21380.pdf?_ga=2.207162320.2143540600.1538477538-2116291175.1538477538> [retrieved on 20181002], DOI: 10.1177/112067210001000205
- CHRISTOPHE BAUDOUIN ET AL: "Role of Hyperosmolarity in the Pathogenesis and Management of Dry Eye Disease: Proceedings of the OCEAN Group Meeting", OCULAR SURFACE, vol. 11, no. 4, 1 October 2013 (2013-10-01), pages 246 - 258, XP55254960, ISSN: 1542-0124, DOI: 10.1016/j.jtos.2013.07.003
- ALAN TOMLINSON ET AL: "Effectiveness of Dry Eye Therapy Under Conditions of Environmental Stress", CURRENT EYE RESEARCH, vol. 38, no. 2, 7 January 2013 (2013-01-07), US, pages 229 - 236, XP55593415, ISSN: 0271-3683, DOI: 10.3109/02713683.2012.757323

## Beschreibung

### GEBIET DER ERFINDUNG

Die Erfindung betrifft eine Zusammensetzung, wie in den Patentansprüchen definiert, zur Vorbeugung und Behandlung des Trockenen Auges oder von Benetzungsstörungen der Hornhaut (Cornea) des Auges.

### HINTERGRUND DER ERFINDUNG

Das Syndrom des trockenen Auges, auch bekannt als Keratokonjunktivitis sicca, ist eine chronische und üblicherweise fortschreitende Erkrankung, die durch mehrere Ursachen hervorgerufen und mit einer Reihe von unterschiedlichen Symptomen in Verbindung gebracht wird. Neuerdings erhält die Wissenschaft mehr und mehr Einblick in die genaue Pathogenese der Erkrankung bis hin zu den Entstehungsmechanismen auf molekularbiologischem Level. Nach gegenwärtigem Kenntnisstand gibt es eine Reihe von Mechanismen, auf die die Entstehung der Krankheit zurückgeführt wird (Michael A. Lemp: The Definition and Classification of Dry Eye Disease: Report of the Definition and Classification Subcommittee of the International Dry Eye Work Shop 2007. The Ocular Surface, April 2007, Vol. 5, no. 2). Diese umfassen einen Rückgang oder vollständige Aufhebung der Tränenfilmsekretion, eine Instabilität des resultierenden Tränenfilms, die Hyperosmolarität des Tränenfilms, eine Verschlechterung der Tränenfilmaufrisszeit (TBUT), ein Einsetzen von Entzündungsprozessen (Expression von entzündungsmediierenden Zytokinen wie Interleukin-1, Tumornekrosefaktor alpha sowie Matrixmetalloproteinasen), eine Störung der Funktion der Meibom-Drüsen und einen Vitamin-A-Mangel in den entsprechenden Regionen.

Die Symptome der betroffenen Patienten, die sich als therapeutische Angriffspunkte eignen, können eine Störung der visuellen Funktion und Sehkraft (bis hin zu schwerer und stark einschränkender Ausprägung), eine Anfärbbarkeit der Hornhaut in der medizinischen Diagnostik (bis hin zu punktuellen Erosionen), eine Verschlechterung der Funktion der Meibomdrüsen (bis hin zur Keratisierung der Drüsen), eine Verschlechterung der Tränenfilmaufrisszeit (verkürzt bis zum sofortigen Einsetzen des Tränenfilmaufrisses) sowie eine Verschlechterung des Schirmer-Tests (Distanz verkürzt bis zu ≤ 2 mm / 5 min) umfassen.

Die gegenwärtige Behandlung des Syndroms des Trockenen Auges erfolgt stufenweise, wobei das Hauptaugenmerk insbesondere in der Selbstmedikation auf Tränenersatzmitteln liegt. Diese enthalten jeweils unterschiedlich starke Gelbildner, die von Hyaluronsäure (Hylo-Vision^{®} Palette), über Celluloseether und Carbomer in variierenden Konzentrationen reichen.

Ungeachtet der unbestrittenen Wirksamkeit dieses Ansatzes gibt es jedoch Schwachstellen, die unter anderem in den folgenden Punkten bestehen. Zunächst besteht mit den wässrigen Systemen keine zufriedenstellende Behandlungsmöglichkeit eines Vitamin-A-Mangels. Vitamin A ist schlecht wasserlöslich und besser wasserlösliche Vitamin A-Derivate zeigen eine gesteigerte Toxizität, vgl. auch Veröffentlichung durch Myhre AM, Carlsen MH, Bøhn SK, Wold HL, Laake P, Blomhoff R. Water-miscible, emulsified, and solid forms of retinol supplements are more toxic than oil-based preparations. Am J Clin Nutr. 2003 Dec;78(6):1152-9.).

Die Symptome des Syndroms des trockenen Auges in seiner hyperevaporativen Form, das durch eine gestörte oder unzureichende Lipidschicht auf dem Tränenfilm gekennzeichnet ist, kann bei Verwendung rein wässriger Augentropfen nur durch sehr häufigen Gebrauch ausgeglichen werden, was fast zwangsläufig zu Problemen mit der Compliance führt.

Auch viele weitere in der menschlichen Träne physiologisch vorkommende Substanzen können mit einem Gelbildner in wässriger Formulierung nicht ausreichend substituiert werden. Dieser Umstand ist insbesondere deshalb unbefriedigend, da sich aufgrund von positiven Beobachtungen mit autologen Serumaugentropfen, die aus Patientenmaterial hergestellt werden, erhebliche positive Effekte bei der Behandlung des Syndroms des Trockenen Auges erwarten ließen, wenn derartige Substanzen in einer entsprechenden Zubereitung vorhanden wären.

Insbesondere werden weitere, in der menschlichen Träne physiologisch vorkommende Substanzen mit Mitteln des Standes der Technik nicht substituiert, wobei häufig anstelle von in der menschlichen Träne physiologisch vorkommenden Substanzen Komponenten eingesetzt werden, die z.B. synthetischer Natur (Polyvinylpyrrolidon bzw. Polyvinylalkohol) oder gar bakterieller Herkunft sind. So wird das Osmoprotektivum Ectoin durch Fermentation aus dem Bakterium Halomonas elongata isoliert (u.a. Publikation der TU Darmstadt von Hansen et al.). Das des Weiteren eingesetzte Disaccharid Trehalose kommt in Insekten und Hefepilzen, nicht aber in Säugern vor (cf. Baudouin et al. Role of Hyperosmolarity in the Pathogenesis and Management of Dry Eye Disease: Proceedings of the OCEAN Group Meeting. The Ocular Surface / October 2013, Vol. 11 No. 4).

Eine Übersicht über die stufenweise angelegten Therapieoptionen, die u.a. die evidenzbasierte Auswahl der angebrachten Medikation im Over-the-Counter (OTC)-Bereich der Apotheke darstellt, bietet die Publikation von Pfister et al. in der Deutschen Apothekerzeitung der Ausgabe 03/2014 ("Entscheidungshilfen für die Therapie des trockenen Auges"). Die Therapieoptionen umfassen dabei die in der nachfolgenden Tabelle 1 vorgestellten Konzepte.

**Tabelle 1**

| Stadium | Wirkstoff(e) | Pharmazeutische Einstufung |
|---|---|---|
| I | Hyaluronsäure + Euphrasia | Pflanzliche Herkunft (Augentrost aus der Familie der Orobanchaceae) |
| I | Polyvinylpyrrolidon | Synthetische Darstellung aus der Acetylenchemie |
| IIa | Carbomer | Synthetisch; kann Restlösemittel und Monomere enthalten; unverträglich mit mehrwertigen Kationen, die physiologisch in der Tränenflüssigkeit vorkommen (z.B. Calcium) |
| IIa | Hypromellose | Synthetisch durch Umsetzen von Cellulose mit Natronlauge, Propylenoxid und Methylchlorid |
| IIa | Hyaluronsäure | Natürlicher Bestandteil des menschlichen Bindegewebes (Glykosaminoglykan) |
| IIa | Hyaluronsäure + Polyethylenglykol | Synthetisch durch Polymerisation von Ethylenoxid |
| IIa | Hyaluronsäure + Carbomer | Carbomer wird synthetisch dargestellt |
| IIa | Hyaluronsäure + Elektrolyte | Kein physiologisch vorkommendes Osmolyt enthalten; Handelspräparate mit Phosphatpuffer, der im Verdacht steht, Ausfällungen an der Kornea hervorzurufen. |
| IIa | Polyvinylalkohol + Polyvinylpyrrolidon | Synthetisch dargestellt; Hydrolyse von Polyvinylacetat |
| IIa | Tamarindensamenpoly saccharide | Pflanzliche Herkunft (Tamarindenbaum aus der Familie der Fabaceae) |
| IIb | Polyethylenglykol + Propylenglykol | Propylenglykol synthetisch durch Hydrolyse von Propylenoxid dargestellt |
| III | Ciclosporin | Immunsuppressivum; isoliert aus den Schlauchpilzen Tolypocladium inflatum (W. Gams) und Cylindrocarpon lucidum (Booth) |
| Zur Nacht | Carbomer + Dexpanthenol | Carbomer wird synthetisch dargestellt |
| Zur Nacht | Vitamin A in lipidhaltiger Grundlage | Lipidhaltige Grundlage enthält Paraffine und Olefine (Rückstände der Erdöldestillation) |
| Lipidersatz | Carmellose + Glycerol | Cellulosebehandlung mit Chloressigsäure |
| Lipidersatz | Paraffin + Propylenglykol + Phospholipid | Paraffin aus Erdöldestillation, Propylenglykol |
| Lipidersatz | Phospholipide + Vitamin A + Vitamin E | Kein physiologisch vorkommendes Osmolyt enthalten |

Wie in der aktuellen Entscheidungshilfe aus der Deutschen Apothekerzeitung zu erkennen ist, beruht der Großteil der im Markt befindlichen Präparate auf Wirkprinzipien, die entweder synthetisch hergestellt werden, nicht in der physiologischen Tränenflüssigkeit vorkommen oder aus Pflanzen bzw. gar aus Pilzen oder Bakterien isoliert werden. Aufgrund der Darstellungsweise dieser Komponenten besteht bei ihnen prinzipiell das Risiko, vom humanen Immunsystem als körperfremd erkannt zu werden sowie Reste wie Monomere oder Verunreinigungen wie Lösungsmittel, etc. aus der Synthese zu enthalten. Es besteht auch der Bedarf, die Wirksamkeit der Mittel des Standes der Technik zu verbessern.

EP2783695 A1 betrifft ein Medikament in Form von Augentropfen oder Gel zur Behandlung des Trockenen Auges. Das Medikament kann Taurin enthalten.

Es ist eine Aufgabe der vorliegenden Erfindung, die oben angeführten Unzulänglichkeiten zu überwinden und eine verbesserte Zusammensetzung zur Vorbeugung gegen und/oder zur Therapie des Syndroms des trockenen Auges und von Benetzungsstörungen der Cornea bereitzustellen.

### ZUSAMMENFASSUNG DER ERFINDUNG

Zur Lösung dieser Aufgabe stellt die vorliegende Erfindung bereit:
Eine Zusammensetzung wie in Anspruch 1 definiert zur Vorbeugung und/oder zur Behandlung des Trockenen Auges oder von Benetzungsstörungen der Hornhaut (Cornea) des Auges, wobei die Zusammensetzung einen oder mehrere Osmolyte enthält. Bevorzugte Ausführungsformen sind in den weiteren Ansprüchen definiert.

### DETAILLIERTE BESCHREIBUNG DER ERFINDUNG

Die Erfindung stellt eine Zusammensetzung wie in den Ansprüchen definiert zur Verwendung bei der Behandlung und Vorbeugung (vorzugsweise Behandlung) des Trockenen Auges oder von Benetzungsstörungen der Hornhaut (Cornea) des Auges bereit, wobei die Zusammensetzung einen oder mehrere Osmolyte enthält. Die Zusammensetzung der Erfindung enthält eine oder mehrere lipophile Komponenten und eine wässrige Lösung, die einen oder mehreren Osmolyte enthält.

Die lipophilen Komponenten können eine lipophile Grundlage wie eine Salbengrundlage sein. Die Zusammensetzung wie in den Ansprüchen definiert ist ein ZweiPhasen-System mit einer lipophilen Phase (wie eine Lipidphase) und einer wässrigen Phase, wobei der oder die Osmolyte bevorzugt in der wässrigen Phase enthalten sind. Die Zusammensetzung kann eine Emulsion sein. Die Zusammensetzung wie die Emulsion kann eine wässrige kontinuierliche Phase und eine dispergierte lipophile Phase aufweisen, z.B. eine Öl-in-Wasser-Emulsion. Vorzugsweise weist die Zusammensetzung wie die Emulsion jedoch eine kontinuierliche lipophile Phase und eine dispergierte wässrige Phase auf, wie bei einer Wasser-in-Öl-Emulsion.

Lipophile Komponenten (Verbindungen), die in der Zusammensetzung verwendet werden können, sind Lipide und andere lipophile Komponenten. Diese Komponenten sind dem Fachmann bekannt. Geeignete lipophile Komponenten sind zum Beispiel Wollwachs (Adeps Lanae) oder Wollwachsersatz, Paraffinum subliquidum, Paraffinum subliquidum, Paraffinum perliquidum, Vaseline (z.B. weiße Vaseline), und Lipide (z.B. natürliche pflanzliche Öle) wie Triglyceride (Triacylglycerole, Fette), Phospholipide, Steroide, Castoröl, Carotinoide, Fettsäuren und Wachse (Fettsäurealkylester), die einzeln oder in Kombination zweier oder mehrerer verwendet werden können. Die Phospholipide können Phosphoglyceride (z.B. Lecithine) und/oder Sphingomyeline sein. Die Acylgruppen der Triglyceride, Phospholipide und Wachse, und die Fettsäuren können gesättigt oder ungesättigt sein. Die Anzahl der Kohlenstoffatome (Kettenlängen) der Acylgruppen der Triglyceride, Phospholipide und Wachse und der Fettsäuren kann von 6 bis 30, vorzugsweise von 14 bis 24 und bevorzugt von 16 bis 22 sein. Allgemein können die Acylgruppen und die Fettsäuren mittelkettig (6 bis 12 Kohlenstoffatome) oder langkettig (14 bis 30 Kohlenstoffatome) sein, wobei letztere bevorzugt sind. Beispiele für geeignete Phosphoglyceride sind 2-Dioleoyl-sn-glycero-3-phosphocholin (DOPC) und 1,2-Dipalmitoyl-sn-glycero-3-phosphoglycerol (DPPG). Ein kommerziell erhältliches Gemisch hieraus ist z.B. Softisan^{®}.

Die Zusammensetzung enthält mindestens 60 Gew.-%, bevorzugter mindestens 70 Gew.-% und am bevorzugtesten mindestens 80 Gew.-% lipophile Komponenten enthalten. Der ausgeprägte Anteil an lipophilen Komponenten gleicht effektiv eine Störung der Lipidschicht im Tränenfilm aus. Dies ist insbesondere bei der Behandlung des Syndroms des trockenen Auges in seiner hyperevaporativen Form von Vorteil.

Die Zusammensetzung kann eine Salbe oder eine Creme sein. Solche Formulierungen sind dem Fachmann im Prinzip bekannt, zum Beispiel von dermatologischen Präparaten. Da die Zusammensetzung eine Salbe oder Creme ist, ist der Gehalt an lipophilen Komponenten (Verbindungen) mindestens 60 Gew.-%, bevorzugter mindestens 70 Gew.-% und am bevorzugtesten mindestens 80 Gew.-%.

Die Zusammensetzung kann, insbesondere für Emulsionen, je nach Bedarf Emulgatoren enthalten, um Zusammensetzung oder Emulsion zu stabilisieren. Emulgatoren für pharmazeutische Zusammensetzungen sind dem Fachmann bekannt. Zum Beispiel können Emulgatoren verwendet werden, die in dermatologischen Präparaten verwendet werden. Auch einige der oben genannten Lipide können als Emulgatoren wirken, wie Phospholipide und Fettsäuren. Andere geeignete Emulgatoren sind Kolliphor^{®} EL (BASF), Macrogol 15-Hydroxystearat, und Cetylstearylalkohol. Wenn Emulgatoren vorhanden sind, werden diese zur Bestimmung des Gehalts an lipophilen Komponenten mit eingerechnet.

Die lipophile Grundlage oder die lipophile Phase kann ferner weitere lipophile Bestandteile wie Vitamin A, Tocopherole oder Tocotrienole enthalten. Das Vitamin A kann Retinal, Retinol, Retinsäure, Retinylpalmitat, 3-Dehydroretinol und/oder 3-Dehydroretinal sein. Ein Gehalt an Vitamin A wirkt einem Mangel an Mucin und Defekten in der Mucin-Schicht entgegen. Dieser wird durch die Dysfunktion der Becherzellen hervorgerufen, was wiederum durch einen Mangel dieser essentiellen Substanz verursacht wird (vgl. Karen Brian Fernandez A Stepwise Approach to Treatment of Dry Eyes Published in Primary Care Expert Opinion / Interview May 23, 2013).

Die Zusammensetzung der Erfindung enthält neben den lipophilen Komponenten eine wässrige Lösung. Die wässrige Lösung enthält zumindest Wasser und einen oder mehrere Osmolyte. Die wässrige Lösung kann weitere wasserlösliche pharmazeutische Hilfsstoffe enthalten. Die Osmolyte schützen gegen eine Störung der wässrigen Phase des Tränenfilms, die zur Hyperosmolarität führen und anschließend eine entzündliche Kaskade auslösen kann. Da andererseits die lipophilen Komponenten die Lipidschicht des Tränenfilms schützen, weist die Zusammensetzung der Erfindung eine außergewöhnliche Wirkung zum Schutz des Tränenfilms und zur Behandlung des Trockenen Auges und von Benetzungsstörungen auf.

Der/die Osmolyt/e ist/sind erfindungsgemäß Taurin und/oder Betain (Glycinbetain). Offenbart sind aber auch Ectoin, Sorbitol, Trehalose, Sarcosin, Raffinose, Trimethylamin-N-oxid, Glycerol, Glycin, Propylenglycol, Butylenglycol, Harnstoff, Milchsäure oder Salze derselben wie Natriumlactat, Dicycanamid, Tremella-Extrakt, L-Carnithin, Erythritol, myo-Inositol, Dimethylsulfoniopropionat und Prolin. Es ist auch möglich, die genannten Osmolyte zu kombinieren. Eine bevorzugte Kombination ist die Kombination von Taurin und Betain, eine andere Kombination ist die aus Taurin, Betain und Glycin.

Die Osmolyte können in der Zusammensetzung allgemein in einer Gesamtmenge von 0,1 bis 10 Gew.-%, vorzugsweise 0,2 bis 5 Gew.-%, bevorzugter 0,4 bis 3 Gew.-% an der Gesamtmasse der Zusammensetzung enthalten sein. Je nach dem gewünschten Mischungsverhältnis der Gesamtheit der lipophilen Komponenten, der lipophilen Grundlage oder der lipophilen Phase zum Wassergehalt bzw. zur wässrigen Lösung in der Zusammensetzung, kann der Gehalt der Osmolyte in der wässrigen Lösung entsprechend höher gewählt werden, um nach dem Mischen mit den lipophilen Komponenten den gewünschten Gehalt an Osmolyten in der Zusammensetzung zu erhalten.

Es kann vorgesehen sein, dass Hyaluronsäure in einer Konzentration von 0,05 bis 0,5 % Gew.-%, vorzugsweise von 0,1 bis 0,3 Gew.-% in der Zusammensetzung enthalten ist, wie dies in bekannten, rein wässrigen Systemen, beispielsweise die Präparate Hylo-Vision^{®} HD, Hylo-Vision^{®} HD plus, Hylo-Vision^{®} SafeDrop^{®} und Hylo-Vision^{®} SafeDrop^{®} Gel der Fall ist. Eine von rein wässrigen Systemen abgeleitete, geeignete Konzentration für Butylenglycol and Propylenglykol in der Zusammensetzung liegt zwischen 0,1 und 0,5, vorzugsweise bei etwa 0,3 Gew.-% (Stephen Cohen, Anna Martin, and Kenneth Sall, Evaluation of clinical outcomes in patients with dry eye disease using lubricant eye drops containing polyethylene glycol or carboxymethylcellulose Clin Ophthalmol. 2014; 8: 157-164.) Eine von rein wässrigen Systemen abgeleitete, geeignete Konzentration für Sorbitol in der Zusammensetzung befindet sich im Bereich 1,5 bis 4 Gew.-%, wie es beispielsweise in den kommerziellen Präparaten Vidisic^{®}, Vidisic^{®} Gel, Viscotears^{®}, Viscotears Gel^{®} der Fall ist (zusammengefasst zum Beispiel in: David A. Sullivan,Darlene A. Dartt,Michele A. Meneray. Lacrimal Gland, Tear Film, and Dry Eye Syndromes 2. 1998, page 750). Eine von rein wässrigen Systemen abgeleitete, geeignete Konzentration für Taurin in der Zusammensetzung liegt im Bereich von 0,05 Gew.-% (Fuchs, S.A., Berger,R., Klomp,L.W.J., deKoning,T.J., 2005. D-aminoacids in the central nervous system in health and disease. Mol.Genet.Metab. 85,168-180) bis hin zu 1 oder gar 4 Gew.-%, wie zum Beispiel in kommerziell erhältlichen Augentropfen wie Bestoxol^{®}. Eine von rein wässrigen Systemen abgeleitete, geeignete Konzentration für Betain in der Zusammensetzung liegt zwischen 0,1 Gew.-% und 0,2 Gew.-% (Wei Chen; Xin Zhang; Jinyang Li; Yu Wang; Qi Chen; Chao Hou; Qian Garrett Efficacy of Osmoprotectants on Prevention and Treatment of Murine Dry Eye. Investigative Ophthalmology & Visual Science September 2013, Vol.54, 6287-6297). Eine von rein wässrigen Systemen abgeleitete, geeignete Konzentration für Harnstoff in der Zusammensetzung liegt bei kleiner gleich 0,5 Gew.-%, vorzugsweise zwischen 0,1 und 0,4 Gew.-%, wie zum Beispiel in topischen Anwendungen wie in der von der U.S. Food and Drug Administration (FDA) auf deren Homepage zugänglichen 'Inactive ingredients list' spezifiziert.

In einer bevorzugten Ausführungsformen ist der (sind die) Osmolyt(e) ausgewählt aus der Gruppe bestehend aus Taurin und Betain, und die Zusammensetzung enthält eine Gesamtmenge an diesen Osmolyten von 0,1 bis 10 Gew.-%, vorzugsweise 0,2 bis 5 Gew.-%, bevorzugter 0,4 bis 3 Gew.-% an der Gesamtmasse der Zusammensetzung.

Die wässrige Lösung und die Zusammensetzung kann ferner Hilfsstoffe enthalten, die aus Puffersubstanzen, anorganischen Salzen, organischen Salzen, Anti-Oxidantien, Konservierungsmitteln, Stabilisatoren (z.B. EDTA), Osmolaritätsreglern sowie Mischungen davon besteht. Die wässrige Lösung kann gelöste Elektrolyte enthalten, bei denen es sich vorzugsweise um eine oder mehrere Ionen ausgewählt aus der Gruppe Natriumionen, Kaliumionen, Calciumionen, Magnesiumionen und Chloridionen handelt, und/oder Proteine umfassen, bei denen es sich vorzugsweise um ein oder mehrere Proteine ausgewählt aus der Gruppe Lacritin, Lactoferrin und Serumalbumin, vorzugsweise bovines Serumalbumin handeln kann, und/oder Zucker, vorzugsweise Glucose, und/oder Allantoin und/oder Dexpanthenol umfassen. Des Weiteren kann die Zusammensetzung auch Vitamine wie Vitamin B12, Vitamin A oder Vitamin E enthalten.

In einer bevorzugten Ausführungsform enthält die Zusammensetzung keine antimikrobiellen Konservierungsmittel, insbesondere keine kationischen Tenside als Konservierungsmittel wie Benzalkoniumchlorid, da diese die Cornea schädigen können.

Mögliche Puffer der wässrigen Lösung sind u.a. Boratpuffer, Citratpuffer, Phosphatpuffer, Tris-Puffer, Hydrogencarbonatpuffer sowie Mischungen oder Kombinationen hiervon. Bevorzugte Puffer sind Citrat- und Tris-Puffer, auch in Kombination mit weiteren Puffersalzen. In einer Ausführungsform enthält die wässrige Lösung keinen Puffer. Der pH-Wert der Zusammensetzung kann zwischen 7,0 und 7,4 liegen.

Die Osmolalität der wässrigen Lösung kann, insbesondere bei Vorhandensein eines Puffersystems, auf 100-1000 mOsm/kg, bevorzugt 200-500 mOsm/kg, besonders bevorzugt 220- 350 mOsm/kg eingestellt werden.

Die Zusammensetzung der Erfindung kann nach dem Fachmann bekannten Verfahren durch Mischen der Komponenten hergestellt werden. Es ist z.B. möglich, zuerst Mischungen der lipophilen Komponenten einerseits und der hydrophilen Komponenten andererseits getrennt voneinander herzustellen. Falls die Zusammensetzung zwei oder mehr lipophile Komponenten enthält, können diese gemischt werden, z.B. durch Zusammenschmelzen, oder durch Mischen von Lösungen derselben in einem Alkohol mit einem Siedepunkt von unter 100°C wie Ethanol (der Alkohol wird nachfolgend entfernt). Die wässrige Lösung kann durch Auflösen aller wasserlöslichen Bestandteile in Wasser hergestellt und bei Bedarf der pH-Wert eingestellt werden. Anschließend können die lipophilen Komponenten mit der wässrigen Lösung gemischt werden. Verfahren zur Herstellung von Salben, Cremes, Wasser-in-Öl- und Öl-in-Wasser-Emulsionen sind dem Fachmann bekannt. Liposomenzusammensetzungen und deren Herstellung sind ebenfalls bekannt. Zum Beispiel können die in WO2017074475 und WO2018073720 beschriebenen Liposomen (ohne die dort genannten Wirkstoffe) verwendet werden. Die Zusammensetzung kann steril in geeignete Einzeldosisbehältnisse oder Mehrdosenbehältnisse eingebracht werden. Die Zubereitungen werden auf verschiedene Spezifikationsparameter getestet. Die Zusammensetzungen können eine Viskosität im Bereich von 5.000 bis 300.000 mPa·s bei 25°C haben, bevorzugt eine Viskosität im Bereich von 10.000 bis 200.000 mPa·s bei 25°C.

Die Zusammensetzung der Erfindung wird zur Vorbeugung oder Behandlung des Trockenen Auges oder von Benetzungsstörungen der Hornhaut (Cornea) verwendet, insbesondere des hyperevaporativen Trockenen Auges. Die Vorbeugung oder Behandlung kann am Menschen oder am Tier, vorzugsweise am Menschen durchgeführt werden. Flüssige oder fließfähige Formulierungen können durch Eintropfen in die Augen verabreicht werden. Salben und Cremes können auf die Augenränder und -lider aufgetragen werden. Wegen des vorzugsweise hohen Gehalts an lipophilen Komponenten, kann die Zusammensetzung die Lichtbrechungseigenschaften der Hornhaut und damit die Sehfähigkeit vorübergehend beeinträchtigen. Daher wird die Zusammensetzung vorzugsweise vor einer Pause, während der keine besonderen Anforderungen an die Sehfähigkeit gestellt werden oder vor der Nachtruhe, auf die Augen, insbesondere die Augenränder und -lider, aufgetragen. Durch den hohen Anteil an lipophilen Komponenten ist es nicht erforderlich, die Behandlung oft zu wiederholen. Es kann in einer Anwendungsvariante auch ausreichend sein, die Zusammensetzung ausschließlich vor der Nachtruhe, mithin einmal täglich aufzutragen. Die Behandlung kann fortgeführt werden, solange Symptome des Trockenen Auges oder von Benetzungsstörungen vorliegen.

In einer Ausführungsform ist die Zusammensetzung zur Verwendung in der Behandlung des Syndroms des Trockenen Auges im Rahmen einer Langzeitbehandlung vorgesehen, wobei die Zusammensetzung über einen Zeitraum von mehr als zwei und vorzugsweise mehr als sechs Monaten wenigstens einmal täglich verabreicht wird. In einer Ausführungsform ist die Zusammensetzung zur Verwendung in der Behandlung des Syndroms des trockenen Auges durch Auftragung auf das Auge vor der Nachtruhe vorgesehen. Die der Erfindung zu Grunde liegende Herangehensweise ist äußerst effizient, auch stark ausgeprägte Symptome des Syndroms des trockenen Auges in Langzeittherapie zu überwinden.

Wegen des Gehalts sowohl an lipophilen Komponenten als auch an Osmolyten ist die Zusammensetzung von besonderer Wirksamkeit gegen Benetzungsstörungen des Auges und das Trockene Auges, und kann zur Behandlung und/oder Vorbeugung des hyperevaporativen Trockene Auge verwendet werden. Gleichzeitig spendet die Zusammensetzung wegen der Wasserbindungseigenschaften der Osmolyte Feuchtigkeit, wodurch auch die mittlere wässrige Schicht des Tränenfilms und die der Cornea zugewandte innere Schicht des Tränenfilms stabilisiert werden. Die Tränenfilmaufrisszeiten eines Patienten werden stark verlängert.

Die erfindungsgemäße Zusammensetzung empfindet die Zusammensetzung der Träne mit ihren physiologischen Bestandteilen gut nach. Diese Herangehensweise ist vorteilhaft, weil dadurch fehlende physiologische Bestandteile ersetzt werden können, ohne dass mögliche Nebenwirkungen wie allergische Reaktionen auf synthetische oder bakterielle Substanzen mit in Kauf genommen werden müssen.

Zusammenfassend besteht die erfindungsgemäße Zusammensetzung für eine Verwendung in der Behandlung des Syndroms des Trockenen Auges aus einer lipophilen Grundlage und einer dispersen wässrigen Phase, die eine oder mehr Osmolyte enthält, wobei die Osmolyten bevorzugt in der natürlichen Tränenflüssigkeit zu detektieren sind wie die Inhaltsstoffe Taurin oder Betain. Die Zubereitung kann konservierungsmittelfrei sein. Die Zubereitung ist eine Salbe oder eine Creme. Sie kann zusätzlich die Osmolyte Ectoin, Sorbitol, Trehalose, Sarcosin, Raffinose, Trimethylamin-N-oxid, Glycerol, Glycin, Propylenglykol, Butylenglykol, Milchsäure oder Lactaten wie Natriumlactat, Dicyanamid, Tremella-Extrakt, L-Carnithin, Erythritol, myo-Inositol, Dimethylsulfonioproprionat, Harnstoff oder Prolin einzeln oder in Kombination und in einer adäquaten Konzentration enthalten. Sie kann andere, vergleichbare, insbesondere physiologische, in der menschlichen Tränenflüssigkeit vorkommende Osmolyte enthalten, die oben nicht genannt wurden. Sie kann physiologische Inhaltstoffe in der wässrigen Phase enthalten, die verändert oder unverändert auch in der menschlichen Tränenflüssigkeit vorkommen oder durch analoge Inhaltsstoffe zum Beispiel vom Tier oder synthetisch ersetzt werden können wie zum Beispiel bestimmte Elektrolyte (z.B. Natriumchlorid, Kaliumchlorid, Calciumchlorid und Magnesiumchlorid), Proteine (z.B. Lactoferrin, Serumalbumin), Vitamine oder Glukose. Sie kann eine Kombination der vorgenannten Komponenten in einer therapeutisch anwendbaren Konzentration enthalten.

Einige bevorzugte Ausführungsformen der Zusammensetzung, die offenbart aber nicht beansprucht sind, sind wie folgt:
Zusammensetzung, die eine oder mehrere lipophile Komponenten oder eine lipophile Grundlage und eine wässrige Lösung enthält und die wässrige Lösung einen oder mehreren Osmolyte ausgewählt aus der Gruppe bestehend aus Taurin, Betain, Ectoin, Trimethylamin-N-oxid, Glycin, Harnstoff und Prolin in einer Gesamtenge an diesen Osmolyten von 0,1 bis 10 Gew.-%, vorzugsweise 0,2 bis 5 Gew.-%, bevorzugter 0,4 bis 3 Gew.-% an der Gesamtmasse der Zusammensetzung enthält.

Zusammensetzung, die eine oder mehrere lipophile Komponenten oder eine lipophile Grundlage und eine wässrige Lösung enthält und die wässrige Lösung einen oder mehreren Osmolyte ausgewählt aus der Gruppe bestehend aus Taurin, Betain, Trimethylamin-N-oxid, Glycin, und Prolin in einer Gesamtenge an diesen Osmolyten von 0,1 bis 10 Gew.-%, vorzugsweise 0,2 bis 5 Gew.-%, bevorzugter 0,4 bis 3 Gew.-% an der Gesamtmasse der Zusammensetzung enthält.

Zusammensetzung, die eine oder mehrere lipophile Komponenten oder eine lipophile Grundlage und eine wässrige Lösung enthält und die wässrige Lösung einen oder mehreren Osmolyte ausgewählt aus der Gruppe bestehend aus Taurin, Betain, Trimethylamin-N-oxid, Glycin, Harnstoff und Prolin in einer Gesamtenge an diesen Osmolyten von 0,1 bis 10 Gew.-%, vorzugsweise 0,2 bis 5 Gew.-%, bevorzugter 0,4 bis 3 Gew.-% in der Gesamtmasse der Zusammensetzung enthält, und die Zusammensetzung mindestens 50 Gew.-% an lipophilen Komponenten (Verbindungen) enthält.

Zusammensetzung, die eine oder mehrere lipophile Komponenten oder eine lipophile Grundlage und eine wässrige Lösung enthält und die wässrige Lösung einen oder mehreren Osmolyte ausgewählt aus der Gruppe bestehend aus Taurin, Betain, Trimethylamin-N-oxid, Glycin, Harnstoff und Prolin in einer Gesamtenge an diesen Osmolyten von 0,1 bis 10 Gew.-%, vorzugsweise 0,2 bis 5 Gew.-%, bevorzugter 0,4 bis 3 Gew.-% in der Gesamtmasse der Zusammensetzung enthält, und die Zusammensetzung mindestens 60 Gew.-%, bevorzugt mindestens 70 Gew.-% an lipophilen Komponenten (Verbindungen) enthält.

Diese bevorzugte Ausführungsformen sind mit weiteren bevorzugten oben beschriebenen Merkmalen kombinierbar, darunter mit dem Verwendungszweck zur Vorbeugung und vorzugsweise zur Behandlung des Trockenen Auges oder von Benetzungsstörungen des Auges.

Weitere Einzelheiten und Vorteile der Erfindung ergeben sich aus den nachfolgend dargestellten Ausführungsbeispielen.

### BEISPIELE

Weitere Einzelheiten und Vorteile der Erfindung ergeben sich aus den nachfolgend dargestellten Ausführungsbeispielen. Alle Prozentangaben beziehen sich auf Gewichtsprozent der Gesamtzusammensetzung. Die Beispiele 1 bis 7 betreffen den ersten Aspekt der Erfindung. Die Beispiele 8 bis 13 betreffen den zweiten Aspekt der Erfindung.

### Beispiel 1

Vitamin-A-haltige Salbe in Kombination mit Betain and Propylenglycol zur Prophylaxe oder Behandlung des Syndroms des trockenen Auges gegen Störungen der Lipid-, Mucin- und wässrigen Phase des Tränenfilms:

| **Inhaltsstoff** | **Anteil** | **Funktion** |
|---|---|---|
| Weiße Vaseline | 42,00% | Lipidphase, antievaporativ |
| Paraffin | 30,00% | Lipidphase, antievaporativ |
| Adeps Lanae | 12,47% | Lipidphase, antievaporativ |
| Vitamin-A-Palmitat | 0,03% | Vitaminersatz |
| Dexpanthenol | 2,00% | Tränenfilmstabilisator |
| α-Tocopherolacetat | 0,10% | Antioxidans, Radikalfänger |
| Cetylstearylalkohol | 2,30% | Emulgator |
| Betain | 0,20% | Physiologisches Osmolyt |
| Propylenglycol | 1,00% | Feuchthaltemittel |
| Wasser für Injektionszwecke | 9,90% | Lösungsmittel |

Die Herstellung des Präparates erfolgt nach den anerkannten Regeln der pharmazeutischen Technologie und der Guten Herstellpraxis von Sterilia. So werden die Inhaltsstoffe der Lipidphase (Vaseline, dünnflüssiges Paraffin, Wollwachs oder Wollwachsersatz sowie der entsprechende Emulgator) erhitzt und zusammen geschmolzen. Vitamin A wird hinzugesetzt, in der Mischung homogenisiert und im Anschluss daran wird die erhaltene Mischung sterilfiltriert (0,22 µm Porengröße). Parallel dazu werden die Bestandteile der wässrigen Phase (Dexpanthenol, α-Tocopherolacetat, Betain und Propylenglycol) im bereitgestellten Wasser für Injektionszwecke gelöst und durch einen geeigneten Sterilfilter gegeben. Nach der Durchführung der entsprechenden Inprozesskontrollen werden die Mischungen homogenisiert und aseptisch in sterile Tuben abgefüllt sowie beschriftet.

### Beispiel 2

Vitamin-A-haltige Salbe in Kombination mit Taurin and Sorbitol zur Übernacht-Behandlung des Syndroms des trockenen Auges gegen Störungen der Lipid-, Mucin- und wäßrigen Schicht des Tränenfilms und in Verbindung mit Elektrolyten als natürliche Bestandteile der wäßrigen Phase der menschlichen Träne:

| **Inhaltsstoff** | **Anteil** | **Funktion** |
|---|---|---|
| Weiße Vaseline | 50,0% | Lipidphase, antievaporativ |
| Paraffin | 18,25% | Lipidphase, antievaporativ |
| Softisan^{®} | 16,6% | Lipidphase, antievaporativ |
| Vitamin-A-palmitat | 0,03% | Vitaminersatz |
| Dexpanthenol | 2,0% | Tränenfilmstabilisator |
| α-Tocopherolacetat | 0,1% | Antioxidans, Radikalfänger |
| Cetylstearylalcohol | 2,3% | Emulgator |
| Sorbitol | 1,5 % | Feuchthaltemittel/Osmolyt |
| Taurin | 1,0 % | Humectant / physiologisches Osmolyt |
| Natriumchlorid | 0,2 % | Elektrolyt |
| Kaliumchlorid | 0,1 % | Elektrolyt |
| Calciumchlorid | 0,02 % | Elektrolyt |
| Wasser für Injektionszwecke | 7,9% | Lösungsmittel |

Die Herstellung erfolgt analog zu Beispiel 1.

### Beispiele 3 bis 6

Analog zu Beispiel 1 werden Zusammensetzungen hergestellt, bei denen das Betain durch myo-Inositol, Dimethylsulfoniopropionat und Prolin bzw. Trimethylamin-N-oxid ersetzt ist (Beispiele 3-5). In Beispiel 6 wird das Betain von Beispiel 1 durch 0,1% Betain plus 0,1% Taurin ersetzt.

### Beispiel 7 (nicht erfindungsgemäß)

Emulsion auf der Basis natürlicher Ausgangsstoffe für die Lipidphase. Als Pflanzliches Öl kann z.B. Sonnenblumenkern-, Kokosnuss- oder Rizinusöl dienen.

| **Inhaltsstoff** | **Anteil** | **Funktion** |
|---|---|---|
| Pflanzliches Öl | 2,0 % | Lipidanteil |
| Glycerol | 1,0 % | Feuchthaltemittel |
| Tylaxapol | 0,15 % | Emulgator |
| Poloxamer 188 | 0,05 % | Co-Emulgator |
| Tris | 0,05 % | Puffer |
| Taurin | 0,5% | Osmolyt |
| Wasser | Ad 100,0% | Solvent |

Die Emulsion wird nach den Regeln der pharmazeutischen Wissenschaft hergestellt. Die fettlöslichen Bestandteile werden mittels starkem Rühren in der wässrigen Phase dispergiert. Anschließend wird der pH-Wert sowie das Zetapotential der Emulsion sowie weitere Qualitätsparameter bestimmt und insbesondere der pH bei Bedarf angepasst.

### Beispiel 8 (nicht erfindungsgemäß)

Niedrig konzentriertes Hyaluronsäuregel in Kombination mit Betain zur Prophylaxe des Syndroms des trockenen Auges, insbesondere gegen Störungen der Mucin- und wässrigen Phase des Tränenfilms mit den in der folgenden Tabelle angegebenen Inhaltsstoffen:

| **Inhaltsstoff** | **Anteil** | **Funktion** |
|---|---|---|
| Natriumhyaluronat | 0,10 g | Physiologischer Gelbildner |
| Natriumchlorid | 0,10 g | Elektrolyt |
| Natriumcitrat | 0,10 g | Puffersalz |
| Betain | 0,20 g | Physiologisches Osmolyt |
| Sorbitol | q.s. | Physiologisches Tonizitätsmittel zur Einstellung der Tonizität |
| Citronensäure | pH=7,0 bis 7,4 | pH-Einstellung |
| Wasser für Injektionszwecke | Ad 100 ml | Lösungsmittel |

Die Herstellung des Präparates erfolgt nach den anerkannten Regeln der pharmazeutischen Technologie und der Guten Herstellpraxis von Sterilia. So kann eine Lösung der Salze, Sorbitol und Betain im Wasser für Injektionszwecke homogenisiert und mit dem Gelbildner versetzt werden. Nach dem Lösen dieser Komponente und anschließendem Rühren für ausreichend lange Zeit kann durch Filtration mit einer Porengröße von 0.22 µm sterilisiert werden. Der Sterilfilter besteht dabei aus einem geeigneten Material, das keine Partikel abgibt, selbst mit einer geeigneten Methode sterilisierbar ist und die enthaltenen Bestandteile nicht adsorbiert. Nach der Durchführung der entsprechenden Inprozesskontrollen wird das erhaltene Gel zur Anwendung am Auge aseptisch in sterile Fläschchen mit Tropfer abgefüllt oder einem Blow-Fill-Prozess zum Erhalt von Einzeldosenopthiolen unterworfen sowie beschriftet.

### Beispiel 9 (nicht erfindungsgemäß)

Hochviskoses Augengel mit Taurin sowie physiologischen Elektrolyten zur Behandlung insbesondere von schweren Formen des Syndroms des trockenen Auges mit den in der folgenden Tabelle angegebenen Inhaltsstoffen:

| **Inhaltsstoff** | **Anteil** | **Funktion** |
|---|---|---|
| Natriumhyaluronat | 0,30 g | Physiologischer Gelbildner |
| Natriumcitrat | 0,10 g | Puffersalz |
| Taurin | 0,20 g | Physiologisches Osmolyt |
| Natriumchlorid | 0,10 g | Elektrolyt |
| Kaliumchlorid | 0,10 % | Elektrolyt |
| Calciumchlorid | 0,02 % | Elektrolyt |
| Magnesiumchlorid | 0,20 % | Elektrolyt |
| Sorbitol | q.s. | Physiologisches Tonizitätsmittel |
| Citronensäure | pH=7,0 bis 7,4 | pH-Einstellung |
| Wasser für Injektionszwecke | ad 100 mL | Lösungsmittel |

Die Herstellung erfolgt analog zu Beispiel 7.

### Beispiele 10 bis 13 (nicht erfindungsgemäß)

Analog zu Beispiel 8 werden Zusammensetzungen hergestellt, bei denen das Betain durch Harnstoff, Cholin bzw. Trimethylamin-N-oxid ersetzt ist (Beispiele 10-12). In Beispiel 13 wird das Betain von Beispiel 8 durch 0,1% Betain plus 0,1% Taurin ersetzt.

## Patentansprüche

1. Zusammensetzung zur Verwendung bei der Behandlung oder Vorbeugung des Trockenen Auges oder von Benetzungsstörungen der Hornhaut (Cornea) des Auges, wobei die Zusammensetzung einen oder mehrere Osmolyte enthält,
wobei die Zusammensetzung eine Salbe oder eine Creme ist und eine lipophile Phase und eine wässrige Phase enthält und die wässrige Phase den einen oder die mehreren Osmolyte enthält,
wobei die Zusammensetzung mindestens 60 Gew.-% Gehalt an lipophilen Komponenten enthält und
wobei die Zusammensetzung Taurin und/oder Betain als Osmolyt enthält.

2. Die Zusammensetzung zur Verwendung gemäß Anspruch 1, wobei die Zusammensetzung eine oder mehrere lipophile Komponenten oder eine lipophile Grundlage und eine wässrige Lösung enthält und die wässrige Lösung den einen oder die mehreren Osmolyte enthält.

3. Die Zusammensetzung zur Verwendung gemäß einem der Ansprüche 1 oder 2, wobei die Zusammensetzung eine wässrige kontinuierliche Phase und eine dispergierte lipophile Phase aufweist (Öl-in-Wasser-Emulsion); oder wobei die Zusammensetzung eine kontinuierliche lipophile Phase und eine dispergierte wässrige Phase aufweist (Wasser-in-Öl-Emulsion).

4. Die Zusammensetzung zur Verwendung gemäß einem der Ansprüche 2 oder 3, wobei die Zusammensetzung mindestens 70 Gew.-% und bevorzugt mindestens 80 Gew.-% Gehalt an lipophilen Komponenten (Verbindungen) enthält.

5. Die Zusammensetzung zur Verwendung nach einem der Ansprüche 2 bis 4, wobei die lipophilen Komponenten oder die lipophile Grundlage oder die lipophile Phase lipophile Stoffe enthält, ausgewählt aus der Gruppe bestehend aus Wollwachs, weißer Vaseline, Paraffinum subliquidum, Paraffinum perliquidum, und Lipiden wie Triglyceriden (wie mittelkettigen Triglyceriden), Castoröl, Phospholipiden, Steroiden, Carotinoiden, Fettsäuren und Wachsen.

6. Zusammensetzung zur Verwendung nach einem der Ansprüche 2 bis 5, wobei die lipophile Grundlage oder lipophile Phase Vitamin A umfasst, das vorzugsweise eine oder mehrere Verbindungen ausgewählt aus der Gruppe Retinal, Retinol, Retinsäure, Retinylpalmitat, 3-Dehydroretinol und 3-Dehydroretinal ist.

7. Die Zusammensetzung zur Verwendung nach einem der vorhergehenden Ansprüche zur Verwendung bei der Behandlung des hyperevaporativen Trockenen Auges.

8. Zusammensetzung zur Verwendung nach einem der vorhergehenden Ansprüche, wobei die Zusammensetzung von 0,1 bis 10 Gew.-%, vorzugsweise 0,2 bis 5 Gew.-%, bevorzugter 0,4 bis 3 Gew.-% Osmolyte gemessen an der Gesamtmasse der Zusammensetzung enthält.

9. Einzeldosisbehältnis oder Mehrdosenbehältnis, enthaltend die Zusammensetzung zur Verwendung nach einem der vorhergehenden Ansprüche.

## Claims

1. Composition for use in the treatment or prevention of dry eye or of corneal wetting disorders, wherein the composition contains one or more osmolytes,
wherein the composition is an ointment or a cream and contains a lipophilic phase and an aqueous phase, and the aqueous phase contains the one or more osmolytes,
wherein the composition contains at least 60% by weight of lipophilic components and
wherein the composition contains taurine and/or betaine as osmolytes.

2. The composition for the use according to claim 1, wherein the composition contains one or more lipophilic components or a lipophilic base and an aqueous solution, and the aqueous solution contains the one or more osmolytes.

3. The composition for the use according to any one of claims 1 or 2, wherein the composition has an aqueous continuous phase and a dispersed lipophilic phase (oil-in-water emulsion); or wherein the composition has a continuous lipophilic phase and a dispersed aqueous phase (water-in-oil emulsion).

4. The composition for the use according to claim 2 or 3, wherein the composition contains at least 70% by weight and preferably at least 80% by weight of lipophilic components (compounds).

5. The composition for the use according to any one of claims 2 to 4, wherein the lipophilic components or the lipophilic base or the lipophilic phase contains lipophilic substances selected from the group consisting of lanolin, white petrolatum, paraffinum subliquidum, paraffinum perliquidum, and lipids such as triglycerides (such as medium-chain triglycerides), castor oil, phospholipids, steroids, carotenoids, fatty acids, and waxes.

6. The composition for the use according to any one of claims 2 to 5, wherein the lipophilic base or lipophilic phase comprises vitamin A, which is preferably one or more compounds selected from the group consisting of retinal, retinol, retinoic acid, retinyl palmitate, 3-dehydroretinol and 3-dehydroretinal.

7. The composition for the use according to any one of the preceding claims for use in the treatment of hyperevaporative dry eye.

8. The composition for the use according to any one of the preceding claims, wherein the composition contains from 0.1 to 10% by weight, preferably from 0.2 to 5% by weight, more preferably from 0.4 to 3% by weight of osmolytes measured relative to the total mass of the composition.

9. Single-dose container or multi-dose container containing the composition for the use according to any one of the preceding claims.

## Revendications

1. Composition à usage dans le traitement ou la prévention de la sécheresse oculaire ou de troubles de l'humidification de la cornée (Cornea) de l'œil, dans laquelle la composition contient un ou plusieurs osmolytes,
dans laquelle la composition est une pommade ou une crème et contient une phase lipophile et une phase aqueuse et la phase aqueuse contient un ou plusieurs osmolytes,
dans laquelle la composition contient au moins 60 % en poids de composants lipophiles et
dans laquelle la composition contient de la taurine et/ou de la bétaïne en tant qu'osmolyte.

2. Composition à usage selon la revendication 1, dans laquelle la composition contient un ou plusieurs composants lipophiles ou une base lipophile et une solution aqueuse, et la solution aqueuse contient un ou plusieurs osmolytes.

3. Composition à usage selon l'une des revendications 1 ou 2, dans laquelle la composition présente une phase aqueuse continue et une phase lipophile dispersée (émulsion huile dans l'eau) ; ou dans laquelle la composition présente une phase lipophile continue et une phase aqueuse dispersée (émulsion eau dans l'huile).

4. Composition à usage selon l'une des revendications 2 ou 3, dans laquelle la composition contient au moins 70 % en poids, et de manière préférée au moins 80 % en poids, de composants lipophiles (composés).

5. Composition à usage selon l'une des revendications 2 à 4, dans laquelle les composants lipophiles ou la base lipophile ou la phase lipophile contiennent des substances lipophiles choisies dans le groupe constitué de cire de laine, vaseline blanche, Paraffinum subliquidum, Paraffinum perliquidum et lipides tels que les triglycérides (comme les triglycérides à chaîne moyenne), huile de ricin, phospholipides, stéroïdes, caroténoïdes, acides gras et cires.

6. Composition à usage selon l'une des revendications 2 à 5, dans laquelle la base lipophile ou la phase lipophile comprend de la vitamine A, qui est de préférence un ou plusieurs composés choisis dans le groupe rétinal, rétinol, acide rétinique, palmitate de rétinyle, 3-déhydrorétinol et 3-déhydrorétinal.

7. Composition à usage selon l'une des revendications précédentes pour une utilisation dans le traitement de la sécheresse oculaire par hyperévaporation.

8. Composition à usage selon l'une des revendications précédentes, dans laquelle la composition contient de 0,1 à 10 % en poids, de préférence de 0,2 à 5 % en poids, de manière davantage préférée de 0,4 à 3 % en poids, d'osmolytes, mesurés par rapport à la masse totale de la composition.

9. Récipient monodose ou multidose contenant la composition à usage selon l'une des revendications précédentes.
